# EUROPÄISCHE PATENTSCHRIFT

(11) **EP 1 272 158 B1**
(45) Veröffentlichungstag und Bekanntmachung des Hinweises auf die Patenterteilung: **26.01.2005**
(21) Anmeldenummer: 01943205.3
(22) Anmeldetag: 29.03.2001
(51) Int. Cl.: A61K 7/48, A45D 44/00

(54) **GESICHTSMASKE AUS EINER BAHN MIT LÖSBAR MITEINANDER VERBUNDENEN TEILKOMPONENTEN**
PACK COMPRISED OF FLEXIBLE SUPPORTS SUITED FOR TAKING UP LIQUIDS, CARE SET CONTAINING THIS PACK, METHOD FOR PRODUCING A PACK AND COSMETIC TREATMENT METHOD INVOLVING THE USE OF THIS PACK
MASQUE CONSTITUE D'UNE BANDE DOTEE DE DEUX COMPOSANTS PARTIELS RELIES DE FA ON AMOVIBLE ET PROCEDE DE TRAITEMENT COSMETIQUE DU VISAGE

(30) Priorität: 10.04.2000 DE 10017530
(43) Veröffentlichungstag der Anmeldung: 08.01.2003
(73) Patentinhaber: Dr. Suwelack Skin & Health Care AG, 48727 Billerbeck (DE)
(72) Erfinder: TEWES-SCHWARZER, Petra, 48308 Senden (DE)
(74) Vertreter: Roos, Peter
(86) Internationale Anmeldenummer: PCT/EP2001/003623
(87) Internationale Veröffentlichungsnummer: WO 2001/076550

(56) Entgegenhaltungen:
- EP-A- 0 063 875
- EP-A- 0 094 285
- EP-A- 1 029 892
- WO-A-98/00117
- FR-A- 2 503 561

## Beschreibung

Die Erfindung betrifft eine Maske aus einem flexiblen Träger, ein Pflegeset, welches diese Maske enthält, ein Verfahren zur Herstellung einer derartigen Maske sowie ein Verfahren zur kosmetischen Behandlung unter Verwendung dieser Maske.

Unter einer Maske im Sinne der vorliegenden Erfindung versteht man eine, auf die Körperoberfläche aufgebrachte Maske, beispielsweise eine Gesichtsmaske, eine Augenmaske, eine Lidmaske, eine Brustmaske, eine Halsmaske, eine Dekolletemaske oder eine auf die Körperextremitäten aufgebrachte Maske. Bevorzugt wird erfindungsgemäß unter der Maske eine Gesichtsmaske verstanden.

Unter einer Maske versteht man weiterhin nach allgemeiner Definition ein spezielles Pflegemittel, welches warm oder kalt auf die Haut, insbesondere Gesichtshaut aufgetragen und nach einer gewissen Einwirkzeit wieder entfernt wird. Diese soll die allgemeine Hautpflege unterstützen und ergänzen.

Weiterhin kann sie ggf. biologisch aktive Mittel wie Kosmetika und / oder Pharmazeutika topisch aufbringen, um eine Prävention und kosmetische Pflege der Haut, eine Prophylaxe und Therapie von Dermatosen und eine Therapie von Verletzungen an der Körperoberfläche zu erreichen. Im Falle von therapeutschen Masken verbleibt die Maske über längere Zeit auf der zu therapierenden Körperoberfläche, und wird ganz oder zum Teil durch Körperflüssigkeiten abgebaut.

Derartige Masken auf Basis von gefriergetrockneten Biomatrixes sind Gegenstand der Deutschen Offenlegungsschrift 40 28 622 sowie der Deutschen Offenlegungsschrift 43 28 329. Die hierbei eingesetzten Masken bestehen üblicherweise aus bahnförmigen Materialien, die in etwa DIN A 4 Format aufweisen und bei der Anwendung erst gefaltet und zugeschnitten werden müssen, um die Gesichtsmaske an die Gesichtskonturen anzupassen. Ein derartiges Vorgehen ist für die behandelnde Person allerdings technisch und zeitlich aufwendig.

Der vorliegenden Erfindung liegt somit die Aufgabe zugrunde, eine Maske aus einem flexiblen, zur Aufnahme von Flüssigkeit geeigneten und/oder aufnehmenden Träger, welcher wenigstens aus einer bahnförmigen Komponente 2 besteht, bereitzustellen, welches aus anwendungstechnischer Sicht einfacher gestaltet ist.

Diese Aufgabe wird dadurch gelöst, daß wenigstens eine der vorgenannten Komponenten 2 aus wenigstens zwei Teilkomponenten 4, 5, 6 besteht, die lösbar miteinander verbunden sind 8, 9, 10.

Die vorliegende Erfindung betrifft somit eine Maske aus einem flexiblen, zur Aufnahme von Flüssigkeiten geeigneten und/oder aufnehmenden Träger, welcher aus wenigstens einer bahnförmigen Komponente 2 besteht, die dadurch gekennzeichnet ist, daß wenigstens eine der Komponenten 2 aus wenigstens zwei Teilkomponenten 4, 5, 6 besteht, die lösbar miteinander verbunden sind 8, 9, 10.

Nach einer bevorzugten Ausführungsform der vorliegenden Erfindung weist der Träger wenigstens eine der Komponenten 2 wenigstens eine Öffnung 12, 13 und/oder lösbare Verbindung, 11 auf.

Hierbei ist es bevorzugt, daß die Öffnungen entweder bereits in der Komponente 2 des Trägers selbst vorhanden sind 11, 12 und/oder beim Auflegen auf das Gesicht entstehen 11.

Gemäß einer weiteren bevorzugten Ausführungsform der erfindungsgemäßen Maske weist der Träger eine Dicke von etwa 0,1 mm bis 5 mm, vorzugsweise aber 0,5 mm bis 2,0 mm auf.

Gemäß einer weiteren bevorzugten Ausführungsform weist der Träger auf einer der beiden Oberflächen der Komponenten 2, 3 eine Beschichtung auf. Diese Beschichtung ist mit einer Klebeschicht versehen, durch die die Beschichtung auf dem Träger fixiert wird. Die Klebeschicht kann so gestaltet sein, daß die Beschichtung bei der Anwendung, d.h. bei Kontakt mit Flüssigkeit fest mit dem Träger verbunden bleibt oder sich die Beschichtung vom Träger nach einer gewissen Zeit ablöst. Beschichtung und Klebeschicht bestehen aus einem die Haut nicht irritierenden Material und bilden auf der Träger abgewandten Seite eine Rückenschicht, um einerseits das Handling der Maske zu vereinfachen und um andererseits einen zusätzlichen Okklusionseffekt, durch den z. B. die Penetration von Wirkstoffen, welche zuvor in den Träger inkorpuriert worden sind, in die Haut erhöht werden kann, zu erzeugen. Durch Okklusion wird zudem eine stärkere Hydratation der Haut erreicht.

Als Beschichtungsmaterialien können z.B. die im Sonderdruck des Bundesgesundheitsblatt (39. Jahrgang, März 1996, Nummer 3, Sonderdruck, herausgegeben vom Robert-Koch-Institut - Bundesinstitut für Infektionskrankheiten und nicht übertragbare Krankheiten, Bundesinstitut für gesundheitlichen Verbraucherschutz und Veterinärmedizin, Bundesinstitut für Arzneimittel und Medizinprodukte und Institut für Wasser-, Boden- und Lufthygiene (Umweltbundesamt) "Hinweise zur Beurteilung von Hygienepapieren und Hinweise zur Beurteilung von Intimhygieneerzeugnissen") erwähnten Materialien eingesetzt werden. Dabei handelt es sich im einzelnen z.B. um Polypropylen, Polyethylen, Viskose, Polyester, Avivagen, Zellstoff, Holzstoff, Baumwolle, Polyurethan.

Darüber hinaus kommen auch noch Folien tierischen Ursprungs, z.B. Kollagenfolien oder Folien biotechnologischen Ursprungs z.B. Paramylonfolie in Betracht.

Die Klebeschicht besteht beispielsweise aus thermoplastischem Kautschuk, Harzen, Wachsen und Ölen oder Fibrin. Alternativ wird die vorgenannte Schicht in an sich bekannter Weise aufgetragen, z.B. durch Aufsprühen einer Lösung oder Dispersion eines Polymeren. Schließlich kann die Schicht am Träger auch mechanisch befestigt werden, durch Rändeln, Nadeln oder ähnliche Verfahren.

Nach einer weiteren bevorzugten Ausführungsform enthält der Träger der erfindungsgemäßen Maske im wesentlichen eine Biomatrix pflanzlichen und/oder tierischen Ursprungs.

Unter einer Biomatrix pflanzlichen Ursprungs versteht man sowohl natürliche Polysaccharide, beispielsweise auf Basis von Pektinen, Alginaten, Carrageen, Agar und Johannisbrotkernmehl als auch modifizierte Polysaccharide, beispielsweise auf Basis von Cellulosederivaten wie Celluloseäthern. Bezüglich weiterer Materialien dieser Biomatrixes auf Pflanzenmaterialien verweisen wir auf die Deutsche Offenlegungsschrift 43 28 329.

Nach einer bevorzugten Ausführungsform der vorliegenden Erfindung enthält die erfindungsgemäße Maske einen Träger auf Basis von Kollagen, also tierischen Ursprungs. Bezüglich näherer Informationen hierzu verweisen wir beispielsweise auf die Deutsche Offenlegungsschrift 40 28 622 sowie auf die Deutsche Patentschrift 32 03 957.

Nach einer weiteren bevorzugten Ausführungsform enthält der Träger in der erfindungsgemäßen Maske weiterhin biologisch wirksame Stoffe sowie Hilfs- und Zusatzstoffe. Diese Stoffe sind an und für sich bekannt, wir verweisen beispielsweise auf die Deutsche Offenlegungsschrift 43 28 329 und dort Spalte 3, Zeilen 5 bis Spalte 6, Zeile 55.

Nach einer weiteren bevorzugten Ausführungsform handelt es sich bei der erfindungsgemäßen Maske um eine therapeutische oder kosmetische Maske.

Der vorliegenden Erfindung liegt weiter die Aufgabe zugrunde, ein Pflegeset, enthaltend eine Maske nach vorgenannten Ansprüchen bereitzustellen.

Unter Flüssigkeit im Sinne des vorgenannten kosmetischen Behandlungsverfahrens versteht man sowohl hydrophile Flüssigkeiten auf Basis von Wasser wie auch alkoholische Lotionen oder schließlich auch lipophile Flüssigkeiten, beispielsweise pflanzliche oder synthetische Öle.

Ein derartiges erfindungsgemäßes Pflegeset kann neben der Maske eine in der kosmetischen Pflege verwendete, Wasser enthaltende Flüssigkeit oder eine Aktivlotion oder ein Gel enthalten. Weitere mögliche Bestandteile eines derartigen Pflegesets können Wirkstofflösungen, z.B. in Ampullenform, Schwämmchen oder Kosmetikpinsel sein.

Die vorliegende Erfindung wird nunmehr durch Figuren näher erläutert anhand einer Gesichtsmaske.

Es zeigen:
Figur 1, eine erste bevorzugte Ausführungsform der erfindungsgemäßen Maske,
Figur 2, eine weitere bevorzugte Ausführungsform der erfindungsgemäßen Maske.

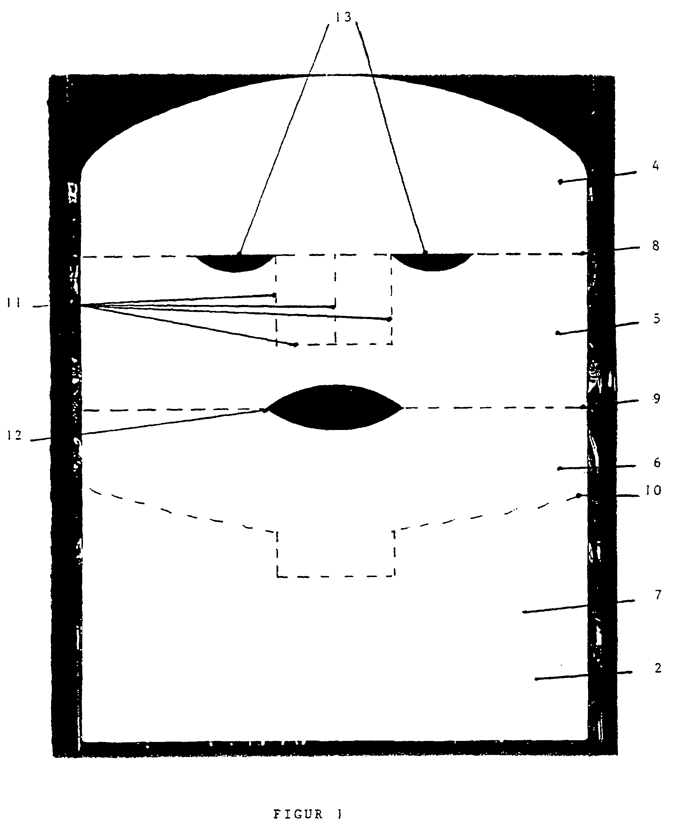

Bei der Maske gemäß Figur 1 besteht der aus einer bahnförmigen Komponente 2 bestehende Träger aus mehreren Teilkomponenten, nämlich dem Stirnteil 4, dem Wangenteil 5, dem Kinnteil 6 und dem Halsteil 7, die zunächst miteinander lösbar verbunden sind. In der Komponente 2 sind bereits Ausstanzungen für den Mund 12 sowie für die Augen 13 vorgesehen, und die Trennung von Stirnteil und Wangenteil wird an der Perforation 8 vorgenommen, die Trennung von Wangenteil und Kinnteil an der Perforation 9 sowie die Trennung des Kinnteils und des Halsteils an der Perforation 10.

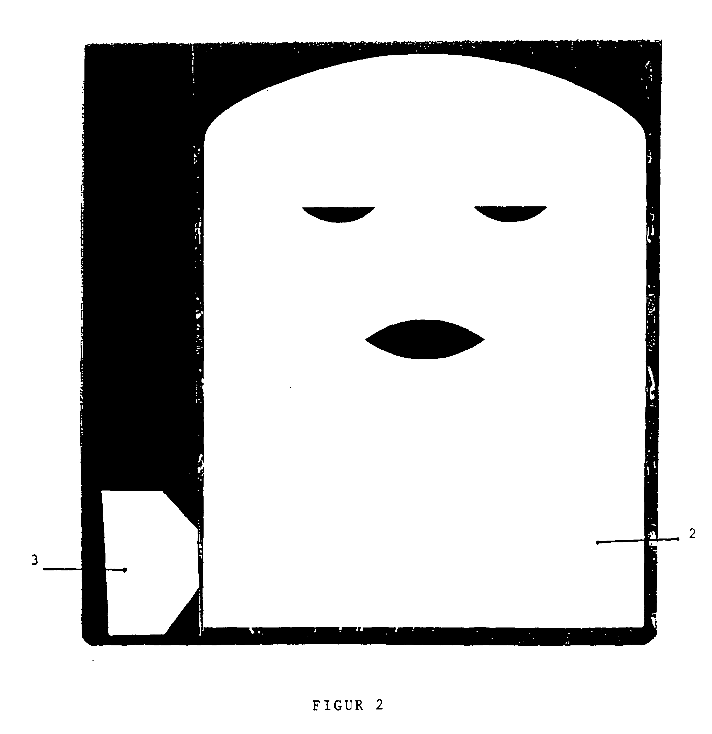

Figur 2 zeigt als weitere bevorzugte Ausführungsform der Maske mit der Komponente 2 (nicht in allen Einzelheiten dargestellt) sowie der Komponente 3, die hier als Nasenteil ausgebildet ist.

Der vorliegenden Erfindung liegt weiterhin die Aufgabe zugrunde, ein Verfahren zur Herstellung einer vorgenannten Maske bereitzustellen. Ein derartiges Verfahren zur Herstellung der erfindungsgemäßen Maske der vorstehenden Art beinhaltet das Bereitstellen von gegebenenfalls mit einer Beschichtung versehenen Komponenten 2, das Vorsehen von wenigstens einem Schwächungsstreifen 8, 9, 10 auf der Komponente 2 sowie gegebenenfalls das Vorsehen von Öffnungen 11, 12, 13 in der Komponente.

Der vorliegenden Erfindung liegt schließlich die Aufgabe zugrunde, ein Verfahren zur kosmetischen Behandlung der Körperoberfläche unter Verwendung einer Maske der vorstehenden Art bereitzustellen.

Gegenstand ist daher schließlich ein Verfahren zur kosmetischen Behandlung der Körperoberfläche unter Verwendung einer Maske der vorgenannten Art, umfassend
(a) die Abtrennung der lösbar verbundenen Teilkomponente 4, 5, 6 aus der Komponente 2,
(b) das Kontaktieren der Körperoberfläche mit einer wirksamen Menge einer Flüssigkeit,
(c) das Aufbringen der ersten Teilkomponente auf wenigstens einen Teil der Körperoberfläche und Befeuchten mit Flüssigkeit,
(d) das Aufbringen einer weiteren Komponente auf die Körperoberfläche und
(e) Wiederholen von Schritt (c),
(f) Wiederholung der vorgenannten Schritte (d) bis (e), bis sämtliche Komponenten der Teilkomponenten auf die Körperoberfläche aufgebracht worden sind.

## Patentansprüche

1. Maske aus einem flexiblen, zur Aufnahme von Flüssigkeiten geeigneten und/oder aufnehmenden Träger, welcher aus wenigstens einer bahnförmigen Komponente besteht, **dadurch gekennzeichnet, daß** wenigstens eine der Komponenten aus wenigstens zwei Teilkomponenten besteht, die lösbar miteinander verbunden sind (8, 9, 10).

2. Maske nach Anspruch 1, **dadurch gekennzeichnet, daß** es sich um eine Gesichtsmaske handelt.

3. Maske nach Anspruch 1 oder 2, **dadurch gekennzeichnet, daß** wenigstens eine der Komponenten (2) des Trägers wenigstens eine Öffnung (11, 12, 13) aufweist.

4. Maske nach Anspruch 1 oder 2, **dadurch gekennzeichnet, daß** die Öffnungen entweder bereits in der Komponente (2) des Trägers selbst vorhanden sind (12, 13) und/oder beim Auflegen auf die Körperoberfläche entstehen (11).

5. Maske nach vorstehenden Ansprüchen, **dadurch gekennzeichnet, daß** der Träger eine Dicke von etwa 0,1 mm bis 5 mm, vorzugsweise 0,5 mm bis 2 mm aufweist.

6. Maske nach vorstehenden Ansprüchen, **dadurch gekennzeichnet, daß** der Träger auf einer der beiden Oberflächen der Komponenten eine Beschichtung aufweist.

7. Maske nach einem der vorstehenden Ansprüche, **dadurch gekennzeichnet, daß** der Träger im wesentlichen eine Biomatrix pflanzlichen und/oder tierischen Ursprungs enthält.

8. Maske nach vorstehenden Ansprüchen, **dadurch gekennzeichnet, daß** der Träger eine Biomatrix auf Basis von Kollagen enthält.

9. Maske nach vorstehenden Ansprüchen, **dadurch gekennzeichnet, daß** der Träger weiterhin biologisch wirksame Stoffe sowie Hilfs- und Zusatzstoffe enthält.

10. Maske nach vorstehenden Ansprüchen, **dadurch gekennzeichnet, daß** es sich um eine therapeutische oder kosmetische Maske handelt.

11. Pflegeset, enthaltend eine Maske nach Ansprüchen 1 bis 10.

12. Verfahren zur Herstellung einer Maske nach irgendeinem der Ansprüche 1 bis 10 durch
a) Bereitstellen von gegebenenfalls mit einer Beschichtung versehenen Komponenten (2, 3),
b) Vorsehen von wenigstens einem Schwächungsstreifen (8, 9, 10) auf der Komponente (2),
c) gegebenenfalls Vorsehen von Öffnungen (11, 12, 13) in der Komponente.

13. Verfahren zur kosmetischen Behandlung der Körperoberfläche unter Verwendung einer Maske gemäß Ansprüchen 1 bis 10 umfassend
a) die Abtrennung der lösbar verbundenen Teilkomponente (4-6) aus der Komponente (2),
b) das Kontaktieren der Körperoberfläche mit einer wirksamen Menge einer Flüssigkeit,
c) das Aufbringen einer ersten Teilkomponente auf wenigstens einen Teil der Körperoberfläche und Befeuchten mit Flüssigkeit,
d) das Aufbringen einer weiteren Komponente auf die Körperoberfläche, und
e) Wiederholung von Schritt c),
f) Wiederholung der vorgenannten Schritte d) bis e), bis sämtliche Komponenten der Teilkomponenten auf die Körperoberfläche aufgebracht worden sind.

## Claims

1. Mask made up of a flexible support suitable for absorbing liquids, and/or absorbent support, which consists of at least one sheet-like component, **characterized in that** at least one of the components consists of at least two partial components detachably joined together (8, 9, 10).

2. Mask according to claim 1, **characterized in that** it is a face mask.

3. Mask according to claim 1 or 2, **characterized in that** at least one of the components (2) of the support has at least one aperture (11, 12, 13).

4. Mask according to claim 1 or 2, **characterized in that** the apertures are either already present in the component (2) of the support itself (12, 13) and/or are formed when the mask is laid on the body surface (11).

5. Mask according to the preceding claims, **characterized in that** the support has a thickness of about 0.1 mm to 5 mm and preferably of 0.5 mm to 2 mm.

6. Mask according to the preceding claims, **characterized in that** the support has a coating on one of the two surfaces of the components.

7. Mask according to one of the preceding claims, **characterized in that** the support substantially contains a biomatrix of vegetable and/or animal origin.

8. Mask according to the preceding claims, **characterized in that** the support contains a biomatrix based on collagen.

9. Mask according to the preceding claims, **characterized in that** the support also contains biologically active substances as well as auxiliary substances and additives.

10. Mask according to the preceding claims, **characterized in that** it is a therapeutic or cosmetic mask.

11. Care set containing a mask according to claims 1 to 10.

12. Process for the production of a mask according to any one of claims 1 to 10, comprising
a) preparing components (2, 3) optionally provided with a coating,
b) providing at least one line of weakness (8, 9, 10) on the component (2), and
c) optionally providing apertures (11, 12, 13) in the component.

13. Method for the cosmetic treatment of the body surface using a mask according to claims 1 to 10, comprising
a) separating the detachably joined partial component (4-6) from the component (2),
b) bringing the body surface into contact with an active amount of a liquid,
c) applying a first partial component to at least part of the body surface and moistening it with liquid,
d) applying another component to the body surface,
e) repeating step c), and
f) repeating steps d) to e) above until all the components of the partial components have been applied to the body surface.

## Revendications

1. Masque en un support flexible, approprié pour accepter et/ou admettant des liquides, qui consiste en au moins un composant en bande, **caractérisé en ce qu'**au moins un des composants consiste en au moins deux composants partiels, qui sont reliés l'un à l'autre de manière amovible (8, 9, 10).

2. Masque selon la revendication 1, **caractérisé en ce qu'**il consiste en un masque pour le visage.

3. Masque selon la revendication 1 ou 2, **caractérisé en ce qu'**au moins des composants (2) du support présente au moins une ouverture (11, 12, 13).

4. Masque selon la revendication 1 ou 2, **caractérisé en ce que** les ouvertures sont déjà présentes dans les composants (2) du support (12, 13) et/ou se forment lors de la pose sur la surface du corps (11).

5. Masque selon les revendications précédentes, **caractérisé en ce que** le support présente une épaisseur allant d'environ 0,1 mm à 5 mm, de préférence de 0,5 mm à 2 mm.

6. Masque selon les revendications précédentes, **caractérisé en ce que** le support présente sur une des deux surfaces des composants, un revêtement.

7. Masque selon les revendications précédentes, **caractérisé en ce que** le support contient essentiellement, une biomatrice d'origine végétale et/ou animale.

8. Masque selon les revendications précédentes, **caractérisé en ce que** le support contient une biomatrice à base de collagène.

9. Masque selon les revendications précédentes, **caractérisé en ce que** le support contient d'autre part, des substances biologiquement actives, ainsi que des auxiliaires et additifs.

10. Masque selon les revendications précédentes, **caractérisé en ce qu'**il s'agit d'un masque thérapeutique ou cosmétique.

11. Trousse de soin, contenant un masque selon les revendications 1 à 10.

12. Procédé de préparation d'un masque selon l'une quelconque des revendications 1 à 10, par
a) préparation des composants (2, 3), le cas échéant munis d'un revêtement,
b) disposition d'au moins une rayure d'affaiblissement (8, 9, 10) sur le composant (2),
c) le cas échéant, disposition d'ouvertures (11, 12, 13) dans les composants.

13. Procédé de traitement cosmétique de la surface du corps en utilisant un masque selon les revendications 1 à 10, comprenant
a) la séparation des composants partiels liés de manière amovible (4-6) depuis le composant (2),
b) la mise en contact de la surface du corps avec une quantité active d'un liquide,
c) l'application d'un premier composant partiel sur au moins une partie de la surface du corps et humidification avec un liquide,
d) l'application d'un autre composant sur la surface du corps, et
e) la répétition de l'étape c),
f) la répétition des étapes d) à e) précédente, jusqu'à ce que tous les composants des composants partiels aient été appliqués sur la surface du corps.
